(19) Europäisches Patentamt
European Patent Office
Office européen des brevets

(11) Numéro de publication : **0 618 194 A1**

(12) # DEMANDE DE BREVET EUROPEEN

(21) Numéro de dépôt : **94400691.5**

(22) Date de dépôt : **30.03.94**

(51) Int. Cl.$^5$ : **C07D 211/70,** A61K 31/445, C07D 211/42

(30) Priorité : **31.03.93 FR 9303783**

(43) Date de publication de la demande : **05.10.94 Bulletin 94/40**

(84) Etats contractants désignés : **AT BE CH DE DK ES FR GB GR IE IT LI LU MC NL PT SE**

(71) Demandeur : **LABORATOIRE L. LAFON**
**19 Avenue du Professeur Cadiot**
**F-94701 Maisons Alfort (FR)**

(72) Inventeur : **Laurent, Philippe**
**37, rue de la Glacière**
**F-69600 Oullins (FR)**

(74) Mandataire : **Le Guen, Gérard et al**
**CABINET LAVOIX**
**2, place d'Estienne d'Orves**
**F-75441 Paris Cédex 09 (FR)**

(54) **3-Phényl-1,2,5,6-tétrahydropyridine, et son utilisation comme sédatif.**

(57) L'invention a pour objet un composé de formule :

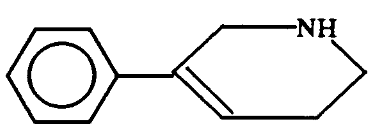

(I)

et ses sels d'addition avec des acides pharmaceutiquement acceptables.
Ces composés sont utiles en thérapeutique comme sédatifs.

EP 0 618 194 A1

Jouve, 18, rue Saint-Denis, 75001 PARIS

La présente invention concerne un nouveau dérivé de l,2,5,6-tétrahydropyridine, son procédé de préparation et ses applications en thérapeutique, notamment comme sédatif.

Dans EP-A-0 192 521, on a déjà décrit des composés de formule :

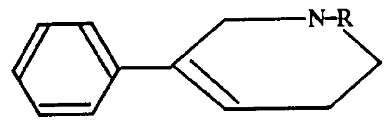

dans laquelle R est un groupe alkyle en $C_2$-$C_4$ et, en particulier, la N-isopropyl-3-phényl-1,2,5,6-tétrahydropyridine possédant des activités sur le système nerveux central, et leur utilisation en thérapeutique comme agents sédatifs.

On a par ailleurs décrit, dans FR-2 416 886, des dérivés de type 3-(3-trifluorométhyl phényl)-1,2,5,6-tétrahydropyridine comme ayant des propriétés anorexigènes.

La présente invention vise à fournir un nouveau dérivé de 1,2,5,6-tétrahydropyridine présentant des propriétés sédatives avec une affinité nettement améliorée pour les récepteurs $\alpha 2$.

La présente invention a plus particulièrement pour objet un composé de formule :

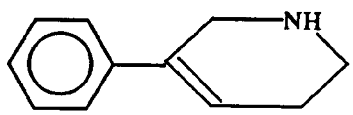 (I)

c'est-à-dire le 3-phényl-1,2,5,6-tétrahydropyridine et ses sels d'addition avec des acides pharmaceutiquement acceptables.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques tels que l'acide formique, l'acide acétique, l'acide propionique, l'acide glycolique, l'acide oxalique, l'acide fumarique, l'acide maléique, l'acide citrique, l'acide malonique, l'acide méthane sulfonique, l'acide lactique, l'acide succinique, l'acide tartrique.

Le composé selon la présente invention peut être préparé par déshydratation d'un composé de formule

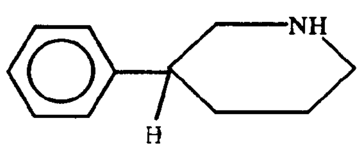 (II)

La déshydratation peut être réalisée notamment à l'aide d'acide paratoluène sulfonique.

Le composé de formule II a été décrit dans EP-A-0 192 521.

Les sels peuvent être obtenus de façon classique par réaction d'un composé de formule I avec un acide pharmaceutiquement acceptable dans un solvant approprié.

L'exemple suivant illustre la préparation un composé selon l'invention.

**EXEMPLE 1** :

**Préparation du 3-phényl-1,2,5,6-tétrahydropyridine, chlorhydrate (CRL 41711)**

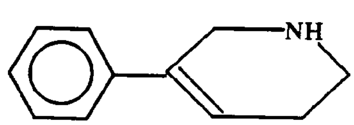 , HCl

On distille l'azéotrope $C_6H_6$-$H_2O$ d'un mélange de 8,3 g (0,047 M) de 3-phényl-3-hydroxypipéridine, 19,72 g (0,104 M) d'acide paratoluènesulfonique monohydrate et 200 ml de $C_6H_6$.

On alcalinise par 30 ml $H_2O$ + 10 ml de lessive de soude, décante le benzène, le lave à l'eau, le sèche sur $MgSO_4$, filtre. On évapore le benzène à sec, reprend à l'éther le résidu, acidifie par l'éthanol chlorhydrique, filtre les cristaux qu'on recristallise dans acétone + éthanol.

On obtient 5 g de produit ayant un point de fusion de 214° C (Rendement = 54 %).

On donnera ci-après des résultats pharmacologiques et toxicologiques mettant en évidence les propriétés intéressantes du composé de formule I :

**I - Prétoxicité** :

Des études de prétoxicité ont été effectuées chez la souris NMRI (3 animaux par dose) avec des doses croissantes de 16, 32, 64, 128, 256 et 512 mg/kg, de produits administrés par voie intrapéritonéale. On donnera la dose provoquant la mort des trois animaux testés.

| Composé | mg/kg IP |
|---|---|
| Exemple 1 | 128 |

**II - Effet sédatif :**

**a) Action sur la motilité spontanée chez la souris**

Une demi-heure après avoir reçu le composé à tester par voie intrapéritonéale, les souris sont placées en actimètre où leur motilité est enregistrée pendant 30 minutes.

Avec le composé de l'exemple 1, on observe une diminution significative de la locomotion dès la dose de 0,032 mg/kg.

**b) Action sur l'agressivité intergroupes**

Après avoir séjourné pendant 3 semaines dans chacune des moitiés d'une cage séparées par une cloison opaque, des groupes de 3 souris reçoivent le composé à tester. Une demi-heure plus tard, les deux groupes d'une même cage sont réunis par retrait de la cloison et on note le nombre de combats qui surviennent en 10 minutes.

Dès la dose de 0,032 mg/kg, mais surtout à la dose de 0,125 mg/kg, le composé de l'exemple 1 diminue le nombre de combats.

**c) Affinité pour les récepteurs $\alpha_2$ du cortex cérébral du rat**

- Méthode :

Les rats (mâles, $CD_1$, Sprague Dawley, 200-250 g) sont sacrifiés par décapitation. Le cortex cérébral est immédiatement prélevé. Les cortex cérébraux de 4 rats sont homogénéisés dans 40 ml de tampon. Les homogénats sont centrifugés à 20.000 tours/minute pendant 15 minutes. Le culot remis en suspension dans 40 ml de tampon est soumis à une deuxième centrifugation (20.000 tours/minute pendant 15 minutes). Le culot ainsi obtenu est mis en suspension dans 8 ml de tampon, puis conservé à -80° C jusqu'à utilisation. Le jour de l'essai, une suspension membranaire est préparée à partir de la suspension congelée. Des aliquots de cette suspension membranaire sont mélangés aux ligands radioactifs (utilisés comme marqueur de chaque type de récepteur) et à des concentrations croissantes du composé à tester, puis mis à incuber (volume final : 1 ml). L'arrêt de la réaction se fait par filtration sur un système HARVESTER 48 trous (bande filtre GF/B WHATMAN). La bande filtre est ensuite lavée 3 fois par 5 ml de tampon puis placée dans un système automatique (BRANDEL) de découpe. Les filtres coupés tombent dans des fioles de comptage et 4 ml de liquide scintillant (Aquasafe 300, ZINSSER) sont distribués automatiquement par le même système (BRANDEL). Chaque échantillon est soumis au comptage de la radioactivité à l'aide d'un compteur à scintillation liquide (KONTRON). Trois séries d'essai sont réalisées avec le composé à tester, chaque essai étant réalisé en double.

La liaison spécifique est définie comme la différence entre la liaison totale et la liaison non spécifique (déplacée par un excès de ligand non radioactif). Les valeurs obtenues en coups par minute (c.p.m.) sont ensuite transformées en désintégrations par minute (d.p.m.) en fonction du rendement du compteur.

Le $CI_{50}$ est défini comme la concentration de la substance étudiée, nécessaire pour déplacer 50 % du marqueur radioactif lié spécifiquement.

Les données expérimentales sont analysées au moyen du logiciel LIGAND* qui calcule la concentration inhibitrice 50 % ($CI_{50}$).

On donnera ci-après les résultats obtenus en utilisant comme marqueur [$^3$H]clonidine.

| Composé testé | CI50 (mole/l) |
|---|---|
| Composé de l'exemple 1 (CRL 41 711) | 2.6 $10^{-8}$ |
| Composé de l'exemple 1 de EP-A-0 192 521 (CRL 41 244) | 70 $10^{-8}$ |

**d) Interaction avec la yohimbine chez la souris**

La yohimbine administrée par voie intrapéritonéale à la dose de 1 mg/kg diminue l'hypomotilité induite par le composé de l'Exemple 1, administré par voie i.p. aux doses de 0,5 et 2 mg/kg.

De même, la yohimbine administrée par voie intrapéritonéale à la dose de 0,5 mg/kg diminue l'hypothermie induite par le composé de l'exemple 1 par voie intrapéritonéale aux doses de 0,125 et 0,5 mg/kg.

La yohimbine étant connue comme un bloqueur des récepteurs $\alpha_2$ noradrénergiques, ces résultats confirment l'action du composé de l'exemple 1 sur les récepteurs $\alpha_2$.

La présente invention a également pour objet des compositions thérapeutiques comprenant à titre de principe actif le composé de formule I ou l'un de ses sels d'addition avec des acides pharmaceutiquement acceptables.

Les compositions thérapeutiques selon l'invention peuvent être administrées chez l'homme ou aux animaux par voie orale ou parentérale.

Elles peuvent être sous la forme de préparations solides, semi-solides ou liquides. Comme exemple, on peut citer les comprimés, les gélules, les suppositoires, les solutions ou suspensions injectables, ainsi que les formes-retard et les formes implantées à libération lente.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

Les composés selon l'invention peuvent être utilisés pour le traitement de l'anxiété, en particulier des états d'anxiété généralisée et des troubles de panique (tels que définis dans DSM III-R) chez l'homme. Dans ces indications, ils peuvent être administrés à des doses journalières de 1 à 100 mg.

## Revendications

1. Composé de formule :

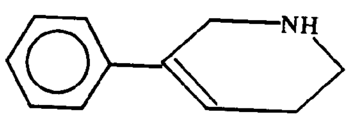

(I)

et ses sels d'addition avec des acides pharmaceutiquement acceptables.

2. Composition thérapeutique comprenant, à titre de principe actif, un composé selon la revendication 1.

3. Procédé de préparation d'un composé selon la revendication 1 comprenant la déshydratation d'un composé de formule :

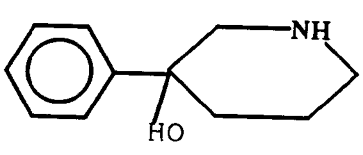

(II)

Office européen des brevets

# RAPPORT DE RECHERCHE EUROPEENNE

Numero de la demande
EP 94 40 0691

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | FR-A-2 576 898 (LAFON) 8 Août 1986<br>* le document en entier * | 1-3 | C07D211/70<br>A61K31/445<br>C07D211/42 |
| D,A | EP-A-0 192 521 (LAFON) 27 Août 1986<br>* le document en entier * | 1-3 | |

DOMAINES TECHNIQUES RECHERCHES (Int.Cl.5)
C07D

Le présent rapport a été établi pour toutes les revendications

| Lieu de la recherche | Date d'achèvement de la recherche | Examinateur |
|---|---|---|
| LA HAYE | 8 Juillet 1994 | Kissler, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)